# EUROPÄISCHE PATENTSCHRIFT

(11) **EP 3 299 026 B1**
(45) Veröffentlichungstag und Bekanntmachung des Hinweises auf die Patenterteilung: **27.05.2020**
(21) Anmeldenummer: 16190132.7
(22) Anmeldetag: 22.09.2016
(51) Int. Cl.: A61K 36/906, A61K 8/04, A61P 17/00, A61K 8/9794, A61Q 19/00

(54) **EXTRAKTE AUS SAMEN VON AFRAMOMUM-ARTEN UND DEREN VERWENDUNG**
EXTRACTS MADE FROM SEEDS OF AFRAMOMUM SPECIES AND THEIR USE
EXTRAITS DE GRAINES D'ESPECES D'AFRAMOMUM ET LEURS UTILISATION

(43) Veröffentlichungstag der Anmeldung: 28.03.2018
(73) Patentinhaber: Dr. Willmar Schwabe GmbH & Co. KG, 76227 Karlsruhe (DE)
(72) Erfinder: KOCH, Egon, 76227 Karlsruhe (DE); VOGEL, Susanne, 76227 Karlsruhe (DE)
(74) Vertreter: Adam, Holger

(56) Entgegenhaltungen:
- EP-A1- 1 800 651
- WO-A1-2005/065701
- WO-A2-2008/064302
- US-A1- 2007 122 500
- US-A1- 2010 055 217
- US-A1- 2010 112 105
- MOHAMMED AMINU ET AL: "Ethyl acetate fraction ofAframomum meleguetafruit ameliorates pancreatic [beta]-cell dysfunction and major diabetes-related parameters in a type 2 diabetes model of rats", JOURNAL OF ETHNOPHARMACOLOGY, Bd. 175, 8. Oktober 2015 (2015-10-08), Seiten 518-527, XP029331835, ISSN: 0378-8741, DOI: 10.1016/J.JEP.2015.10.011
- CHUNG W-Y ET AL: "ANTIOXIDATIVE AND ANTITUMOR PROMOTING EFFECTS OF Ä6Ü-PARADOL AND ITS HOMOLOGS", MUTATION RESEARCH, ELSEVIER, AMSTERDAM, NL, Bd. 496, Nr. 1/02, 20. September 2001 (2001-09-20), Seiten 199-206, XP001119851, ISSN: 0027-5107

## Beschreibung

Die Erfindung betrifft ein Verfahren zur Herstellung von Extrakten aus Samen von *Aframomum melegueta* sowie die so erhältlichen Extrakte und deren Verwendung zur Beseitigung oder Linderung von Juckreiz.

Die menschliche Haut (Cutis) bedeckt mit einer Größe von ca. 2 Quadratmetern fast die gesamte Körperoberfläche. Ihre unterschiedlichen Aufgaben sind der Schutz gegen Pathogene, UV-Strahlung, schädliche Umwelteinflüsse, mechanische Einwirkungen und der Schutz gegen Austrocknung. Eine gesunde und intakte Haut ist von enormer Bedeutung für das Wohlbefinden und das Gefühl, fit zu sein und gut auszusehen.
Der Juckreiz (lat.: Pruritus) ist eine der unbehaglichsten Empfindungen der Haut. Er wird als ein unangenehmes Gefühl, das einen Wunsch zu kratzen auslöst, definiert und kann in akuten (z.B. Insektenstiche, Kontaktallergene) und chronischen Pruritus aufgeteilt werden.
Akuter Juckreiz ist eine physiologische Empfindung auf zahlreiche Reize und ein wichtiges Signal, welches hilft, z. B. Substanzen auf der Haut zu lokalisieren und zu entfernen oder Insekten zu bekämpfen. So hat er eine wichtige Warnfunktion. In Bezug auf Insektenstiche können wir zwei Arten von Reaktionen unterscheiden: eine sofortige Antwort, die vor allem mit der Freisetzung von Histamin verbunden ist und eine verzögerte Immunzell-vermittelte Reaktion, die sich nach 24 bis 48 h entwickelt. Jedoch können die Schwere der Reaktion und der Beitrag der beiden Phasen zu den Symptome bei verschiedenen Individuen sehr variabel ausgeprägt sein.
Wenn Juckreiz länger als 6 Wochen dauert, wird er als chronischer Pruritus bezeichnet. Unerträglicher Juckreiz ist mit vielen Hautkrankheiten verbunden (z.B. Neurodermitis, allergisches Kontaktekzem, allergische Hautausschläge [z. B. durch Arzneimittel oder Nahrungsmittel], Nesselsucht [Urtikaria], Infektionserkrankungen [z. B. Windpocken, Röteln, Masern], Insektenstiche, Parasitenbefall [z. B. Flöhe, Läuse, Milben] und geschädigte, stark belastete Haut, die extrem trocken ist) und stellt ein lästiges Symptom dar. Allerdings ist Pruritus nicht nur ein wesentlicher Bestandteil der Symptome des Patienten in zahlreichen dermatologischen Erkrankungen, sondern kann auch ein Zeichen von einigen systemischen Erkrankungen (z. B. chronische Niereninsuffizienz, Diabetes mellitus, Lebererkrankungen, Durchblutungsstörungen, Krebserkrankungen und HIV-Infektion) sein.
Vergleichbar mit Schmerz, kann chronischer Juckreiz die Lebensqualität dramatisch beeinflussen und macht konzentriertes Arbeiten und erholsamen Schlaf häufig unmöglich. Juckreiz ist ein pathologischer Zustand und führt fast zwangsläufig zum Kratzen, was eine mechanische Schädigung der betroffenen Hautpartien mit sich bringt. Entzündungsmediatoren werden freigesetzt, welche das Jucken noch verstärken und so einen Circulus vitiosus in Bewegung setzen.
Bis vor ein paar Jahren wurde der Juckreiz als unterschwelliger Schmerzreiz wahrgenommen. Erst kürzlich konnte gezeigt werden, dass es eine unabhängige Empfindung der Haut ist, die unabhängig von der Schmerzempfindung auftritt. Es wurden juckreizspezifische Neurone, Mediatoren, spinale Neurone und kortikale Bereiche identifiziert. Oberflächliche Nervenenden in der Haut dienen als Rezeptoren und reagieren auf unterschiedliche Botenstoffe in der Haut und dem Blut und führen zur Stimulation von unmyelinierten Nervenbahnen. Solche Mediatoren sind unter anderem Histamin und Serotonin. Es gibt aber auch noch einige mehr, die entweder direkt die Nervenenden ansteuern (z.B. Interleukin-6, Endothelin) oder die Wirkung von Histamin verstärken (z.B. Prostaglandine).
In Deutschland liegt die Prävalenz von chronischem Pruritus bei etwa 13,5% der Gesamtbevölkerung, wovon die arbeitende Bevölkerung prozentual sogar noch stärker betroffen ist (16,8%). Neuere Studien berichten über eine Inzidenz von 7% pro Jahr.
Die Behandlung des Juckreizes ist so vielfältig wie seine möglichen Entstehungswege. Bis heute gibt es keine einzige universell wirksame Behandlung.
Da die Pathophysiologie des Juckreizes bei den meisten kutanen oder systemischen Erkrankungen unklar bleibt, ist eine antipruriginöse Therapie oft gegen eine Vielzahl von Zielen gerichtet, einschließlich der epidermalen Barriere, dem Immunsystem und dem Nervensystem. Die topische Therapie ist dabei die Hauptstütze der dermatologischen Behandlung bei akutem oder lokalisiertem Jucken oder bei Patienten mit Kontraindikationen für systemische Therapien. Dies umfasst z.B. Feuchtigkeitscremes, Emollentien, Antihistaminika, Kortikosteroide, Immunmodulatoren (z. B. Tacrolimus), Vitamin D3 oder Analoge und Anästhetika.
Bei der Behandlung von Juckreiz sind zunehmend nicht nur effektive, sondern auch sichere neue Naturprodukte gefragt, die die unerwünschten Nebenwirkungen, wie das Problem der "Hautverdünnung", das bei langfristigem Einsatz von potenten topischen Kortisonpräparaten auftreten kann, umgehen.

Es besteht deshalb ein Bedarf nach verbesserten Mitteln zur Behandlung, d.h. Beseitigung oder Linderung von Juckreiz, insbesondere nach Mitteln, welche zu einer langfristigen Beseitigung oder Linderung von Juckreiz führen und weitgehend frei von Nebenwirkungen sind.

Die Aufgabe der vorliegenden Erfindung ist es deshalb, Mittel mit verbesserter juckreizstillender Wirksamkeit zur Verfügung zu stellen, die sich durch eine besondere Verträglichkeit auszeichnen, wenig Nebenwirkungen aufweisen, nicht zur Resistenzbildung neigen und daher zur langzeitigen Anwendung gut geeignet sind. Es wird die Bereitstellung einer beruhigenden Substanz angestrebt, welche den Juckreiz lang anhaltend mindert oder ganz ausschaltet und zugleich die Eigenschaften der Haut verbessert, dabei jedoch den Organismus aufgrund seiner chemischen Eigenschaften nicht belastet. Das Mittel sollte ferner auch einfach in der Handhabung, frei von gesundheitsgefährdenden Substanzen und somit auch für Kinder geeignet sein.

Diese Aufgabe wird erfindungsgemäß gelöst durch die Verwendung von Extrakten aus Samen von *Aframomum melegueta*, die einen neuen und innovativen Ansatz zur Behandlung, d.h. Beseitigung oder Linderung bei akutem und chronischem Juckreiz durch topische Anwendung bieten.

Weitere Aufgabe der Erfindung ist es, solche Extrakte herzustellen, die technologisch vorteilhaft zu topischen Darreichungsformen verarbeitet werden können.

Bestandteil der Erfindung sind ferner Extrakte aus Samen von *Aframomum melegueta*, Verfahren zu deren Herstellung sowie Arzneimittel, Medizinprodukte und Kosmetika zur Linderung von Juckreiz, gekennzeichnet durch einen Gehalt an einem erfindungsgemäßen Extrakt, sowie eine Zubereitung, bestehend aus einem erfindungsgemäßen Extrakt und geeigneten Hilfsstoffen als topische Darreichungsform.
*Aframomum melegueta* ist eine Pflanzenart, die zur Familie der Ingwergewächse (Zingiberaceae) gehört. Die Pflanze ist in Westafrika beheimatet und wird in vielen Gebieten Afrikas kultiviert. Sie wächst als ausdauernde krautige Pflanze mit einem für Ingwergewächse typischen Habitus und erreicht Wuchshöhen von 1,5 Meter. Es wird ein Rhizom als Überdauerungsorgan gebildet. An der Basis der Pflanze werden rosafarbene oder weiße Blütenstände gebildet. Die Blüten sind zwittrig und zygomorph. Es werden bis zu 10 Zentimeter lange Kapselfrüchte gebildet. Die rehroten Samen sind etwa 2 Millimeter groß. Die Droge heißt "Grana paradisi". Die getrockneten Samen, die als Gewürz und Droge verwendet werden, werden auch als Paradieskörner, Guineapfeffer oder Meleguetapfeffer bezeichnet.

In der traditionellen Medizin wird Aframomum als Abführmittel, Galaktagogum, anthelminitisches und blutstillendes Mittel eingesetzt. Es ist auch wirksam gegen Bilharziose. Darüber hinaus wird es bei Darminfektionen eingesetzt, zum Beruhigen bei Verdauungsstörungen und Sodbrennen.

Zudem ist die topische Anwendung von Produkten mit *Aframomum melegueta* Samenextrakt in der kosmetischen Industrie bereits etabliert. Der Extrakt wird schon als Inhaltsstoff von Kosmetika in der EU-Kosmetik Richtlinie aufgelistet, dem hautpflegende Eigenschaften zugeschrieben werden *Aframomum augustifolium* als 70/30 v/v Ethanol/Wasser - Extrakt vermindert z.B. die Fläche oxidierter Proteine und schützt damit die Haut (Patent US 20100055217A1). In einem anderen Patent (Patent US 20070122500A1) wird dem Extrakt aus *Aframomum augustifolium* eine schützende Wirkung vor Hautalterung zugeschrieben.

Aminu et al., J. Ethnopharmacol. 175, S. 518-527 offenbart die Herstellung und Verwendung einer Ethylacetat-Fraktion eines Extraktes aus Samen von *Aframomum melegueta* als Antidiabetikum.

WO 2005/065701 A1 offenbart eine topische Zusammensetzung und ein Verfahren zur Vorbeugung und/oder Linderung von Hautalterungseffekten, umfassend Extrakte von *Aframomum melegueta.*

US 2010/0112105 A1 offenbart die Verwendung von Extrakten von *Aframomum melegueta,* enthaltend eines oder mehrere Phytochemikalien zur Verhinderung des Wachstums von *P. acnes* auf einer Oberfläche.

WO 2008/064302 A2 offenbart ein Verfahren zur Behandlung von erhöhtem Blutzuckerspiegel mit einem Extraktprodukt aus *Aframomum melegueta.*

Des Weiteren offenbart EP 1 800 651 A1 die Extraktion von *Aframomum-melegueta-*Samen für die kosmetische Anwendung.

Des Weiteren offenbart Chung et al., Mutation Research 2001, 496(1), S. 199-206 antioxidative und Antitumor-fördernde Effekte von [6]-Paradol und deren Homologen.

Wir haben nun überraschend beobachtet, dass die erfindungsgemäßen Extrakte aus den Samen von *Aframomum melegueta* über eine Reihe von biologischen Effekten verfügen, die sich für die Behandlung von akutem oder chronischem Juckreiz insbesondere durch topische Anwendung anbieten.
In verschiedenen Studien über Pruritus haben Forscher aufgeklärt, dass Mediatoren und Nervenbahnen an der Juckreizübertragung beteiligt sind. Das Juckreiz-Signal wird hauptsächlich von kleinen Juckreiz-selektiven marklosen C-Fasern mit Ursprung in der Haut übertragen. Daran können Histamin-gesteuerte und nicht Histamin-gesteuerte Neuronen beteiligt sein. In der Haut und den peripheren Nerven gehören dazu als mögliche Mediatoren und Rezeptor-Targets z.B. der H1-Histamin-Rezeptor oder das Enzym Fettsäureamid Hydrolase (engl.: Fatty acid amide hydrolase, FAAH). Pharmakologische Untersuchungen belegen, dass die Extrakte die Freisetzung von Histamin aus Mastzellen blockieren und das Enzym FAAH hemmen, welches Anandamid, ein Endocannabinoid spaltet. Außerdem aktivieren die in den Extrakten enthaltenen Hydroxyalkyl-Ketone die TRPV1-Rezeptoren. Der Extrakt beeinflusst somit mindestens drei verschiedene physiologische Wirkmechanismen, die an der Induktion, der Wahrnehmung und Aufrechterhaltung von Pruritus beteiligt sind. Antioxidative, antimikrobielle und antiinflammatorische Eigenschaften geben bei der topischen Anwendung einen zusätzlichen Vorteil und Nutzen für die Haut.

Die erfindungsgemäßen Extrakte aus Samen von *Aframomum melegueta* können gemäß folgendem Verfahren erhalten werden:
(a) Extraktion von getrockneten und gemahlenen Samen von *Aframomum melegueta* mit einem organischen Lösungsmittel oder mit einem Gemisch aus einem oder mehreren organischen Lösungsmittel und Wasser oder mit Wasser bei einer Temperatur zwischen 10 °C und 100 °C,
(b) Trennung des ausextrahierten Pflanzenmaterials von der Extraktlösung,
(c) ggf. erneute Extraktion des ausextrahierten Pflanzenmaterials mit einem Lösungsmittel gemäß Schritt (a) und Trennung gemäß Schritt (b),
(d) Vereinigung der in den Schritten (b) und (c) gewonnenen Extraktlösungen,
(e) Eindampfen der vereinigten Lösung aus Schritt (d) und Flüssig-flüssig-Verteilung des Eindampfrückstands zwischen Wasser und einem nicht mit Wasser mischbaren organischen Lösungsmittel ausgewählt aus 1-Butanol, 2-Butanon und einem Gemisch aus 2-Butanon und Heptan, um eine wässrige und eine organische Phase zu erhalten,
(f) Trennung der wässrigen und der organischen Phase aus Schritt (e)
(g) Eindampfen und Trocknen der aus (f) erhaltenen organischen Phase zum Trockenextrakt.

Bevorzugte organische Lösungsmittel in Schritt (a) sind Alkohole oder Ketone, wobei der Alkohol vorzugsweise Ethanol und das Keton vorzugsweise Aceton ist. Besonders bevorzugt sind Gemische aus Ethanol bzw. Aceton und Wasser. Das bevorzugte Mischungsverhältnis von Ethanol bzw. Aceton und Wasser beträgt dabei 20 / 80 bis 80 / 20 (Gew./Gew.), besonders bevorzugt 50 / 50 bis 70 / 30. Als Extraktionsverfahren im Schritt (a) kommen bevorzugt Mazeration oder Perkolation in Frage. Die in Schritt (b) beschriebene Trennung des ausextrahierten Pflanzenmaterials von der Extraktlösung erfolgt bevorzugt durch Filtration. Schritt (c) wird in der Regel einmal durchgeführt, ein Verzicht auf Schritt (c) oder mehrmalige Durchführung ist ebenfalls möglich. Mit Wasser nicht mischbare organische Lösungsmittel in Schritt (e) sind Ethylacetat, 1-Butanol, 2-Butanon, sowie Gemische aus 2-Butanon und Heptan, die gegebenenfalls mit Wasser gesättigt sein können. Die Mischung kann hierbei jeweils 60-95 % 2-Butanon, 1-40 % Heptan und 0-10 % Wasser in unterschiedlichen Verhältnissen enthalten. Das Eindampfen und Trocknen in Schritt (g) erfolgt bevorzugt bei einem Druck von 10 - 300 mbar und einer Temperatur von 20 °C - 80 °C. Die Trocknung in Schritt (g) kann durch an sich bekannte Verfahren wie z. B. Gefriertrocknung oder Trocknung im Vakuum bei Raumtemperatur oder erhöhter Temperatur erfolgen. Der in Schritt (g) erhaltene Trockenextrakt enthält entsprechend den Vorgaben des europäischen Arzneibuches einen Wassergehalt von höchstens 5 %.

Bei dem erfindungsgemäßen Verfahren zur Herstellung von Extrakten aus *Aframomum melegueta* werden homogene Extrakte erhalten. Im Gegensatz dazu erhält man beim Eindampfen der vereinigten Extraktlösung aus Schritt (d) ein inhomogenes Zweiphasengemisch, das für die direkte Verarbeitung zu topischen Darreichungsformen wenig geeignet ist. Für eine topische Anwendung soll als Darreichungsform ein hydrophiles Tensidgel, ein wässriges Ethylcellulose-Gel und eine Lotion verwendet werden. Als Hilfsstoffe für das Tensidgel sind Macrogol-Glycerol-Hyroxystearat, Isopropylmyristat, Neutralöl, Propylenglykol und gereinigtes Wasser vorgesehen. Für die Herstellung eines wässrigen Ethylcellulose-Gels werden als Hilfsstoffe Polidocanol, Hydroxyethylcellulose, Glycerol 85%, Xylitol-Lösung 10 % in Wasser, Kaliumsorbat und gereinigtes Wasser verwendet und für die Lotion werden Glycerinmonostearat, Cethylalkohol, Neutralöl, Mandelöl, Lecithin, Glycerol, Xanthan Gummi, Benzylalkohol und gereinigtes Wasser benötigt.

Die erfindungsgemäßen Extrakte können in Form von Gel, Sticks, Cremes, Salben, Rollern, Pflastern oder ähnlichen Zubereitungen topisch verabreicht werden.

Die Dosierung erfolgt dabei so, dass in der topischen Darreichungsform 0.05 Gew.-% bis 10 Gew.-%, bevorzugt 0.2 Gew.-% bis 3 Gew.-% erfindungsgemäßer Extrakt enthalten sind. Typischerweise werden (1-10 mg/cm²) mehrmals täglich, bevorzugt zwei- bis dreimal täglich (2-4 mg/cm²) auf die Haut für die Anwendung beim Menschen aufgetragen.

Die Wirksamkeit der erfindungsgemäßen Extrakte aus den Samen von *Aframomum melegueta* wird durch die nachstehend beschriebenen Versuche belegt.

### Untersuchungen zur Wirksamkeit

### Prüfung auf Hemmung der Histaminfreisetzung aus Mastzellen

Histamin ist ein wichtiger Botenstoff des Menschen und tritt in der Initialphase einer anaphylaktischen (allergischen) Reaktion auf. Histamin ist in fast allen Geweben vorhanden, hauptsächlich wird es in den Granula der Mastzellen und den basophilen Leukozyten gespeichert und wird nach Bedarf freigesetzt. Histamin ist einer der wichtigsten für Juckreiz verantwortlichen körpereigenen Mediatoren. Das Ziel der nachstehend beschriebenen Tests ist es, eine spezifische, anaphylaktische Sofortreaktion zu induzieren, die mit einer Histaminfreisetzung einhergeht und zu prüfen, ob diese durch die erfindungsgemäßen Extrakte gehemmt wird.
Hierfür werden RBL-2H3 basophile Ratten-Leukämiezellen mit einem Immunglobolin (IgE-Anti-DNP) inkubiert. Die Freisetzung von Histamin aus den Zellen wird anschließend durch eine allergische Reaktion mit dem passenden Antigen (DNP-Albumin) ausgelöst. Die Histaminfreisetzung wird mit einem kommerziellen ELISA bestimmt (Kobayashi, M. et al., Int. J. Mol. Med. 2004, 14(5), 879 - 884; Fischer, M. J. et al., Inflamm. Res., 1995, 44, 92 - 97).

Die Zellen wurden in einer 24-Well Testplatte mit einer Zelldichte von 5x10⁵ Zellen/Well ausgesät und der Antikörper mit einer Endkonzentration von 0,02 µg/ml aufgetragen. Zur Messung der spontanen, der spezifischen und der totalen Freisetzung wurden unterschiedliche Wells verwendet. Die Sensibilisierung erfolgte über Nacht (24 Stunden) im Brutschrank bei 37°C und 5% CO₂. Nach der Sensibilisierung wurden die Zellen mit Puffer (NaCl 117 mM, KCl 5,4 mM, MgSO₄ 0,8 mM, CaCl₂ 2,0 mM, Glucose 5,6 mM, Hepes 25 mM, BSA 0,1 %) gewaschen und die erfindungsgemäßen Extrakte zugegeben. Die Zellen wurden mit den Extrakten 30 Minuten im Brutschrank bei 37 °C und 5 % CO₂ inkubiert. Nach der Inkubation der Testsubstanz wurde zur spezifischen Freisetzung das Antigen in einer Endkonzentration von 400 ng/ml und zur spontanen Freisetzung Puffer zugegeben. Die totale Freisetzung wurde durch Zugabe von 2 % Triton X eingeleitet. Die Komponenten inkubierten weitere 30 Minuten im Brutschrank bei 37 °C und 5 % CO₂. Anschließend wurden die Zellen sofort auf Eis gestellt und nach dem Abkühlen 10 Minuten bei 600 x g (4 °C) abzentrifugiert. Der Überstand der Zellen wurde abgenommen und in sterile Reaktionsgefäße überführt. Die Zellen, die mit Triton X behandelt wurden, wurden zusätzlich ultrabeschallt und 10 Minuten bei 1300 x g (4 °C) zentrifugiert. Anschließend wurde der Überstand abgenommen und in sterile Reaktionsgefäße überführt. Der Nachweis von Histamin im Zellüberstand erfolgte mit einem Histamin-ELISA (Firma IBL; Bestellnummer RE59221). Zur Bestimmung wurde der Zellüberstand mit Puffer 1:5 vorverdünnt. Die Messung erfolgte in einem Plattenphotometer (Versamax) bei 450 nm.
Als Positivkontrolle wurde Ketotifen (100 µg/ml) verwendet, als Negativkontrolle 0,1 % DMSO in Puffer.

Die Ergebnisse der Untersuchung sind in Tabelle 1 zusammengestellt. Es ist ersichtlich, dass die erfindungsgemäßen Extrakte aus Aframomum eine eindeutige hemmende Wirkung auf die Freisetzung von Histamin aus RBL-2H3-Zellen ausüben.

**Tabelle 1: Hemmung der Histaminfreisetzung**

| Testsubstanz | Konzentration (µg/ml) | Ergebnis (% Veränderung gegenüber Kontrolle) |
|---|---|---|
| Erfindungsgemäßer Extrakt Bsp. 1 | 50 | -75,88 |
| Erfindungsgemäßer Extrakt Bsp. 2 | 50 | -97,22 |
| Ketotifen | 100 | -80,26 |
| DMSO Kontrolle | 0,1 % | 0 |

### Prüfung auf FAAH-Hemmung

Das Endocannabinoid-System ist ein im Körper allgegenwärtiges Lipid-Signalsystem, das an den unterschiedlichsten regulatorischen Prozessen beteiligt ist.
Einmal in die Zelle eingeschleust werden Endocannabinoide hauptsächlich von zwei Enzymen abgebaut: der Fatty acid amide hydrolase (FAAH) und der Monoacylglycerol lipase (MGL, MAGL).
FAAH ist ein Membranprotein, das bioaktive Amide einschließlich des Endocannabinoids Anandamid in Arachidonsäure und Ethanolamin hydrolisiert und damit die Wirkung von Anandamid beendet. Es ist zudem in der Lage auch andere Endocannabinoide in freie Fettsäuren und Ethanolamin zu zersetzen. Für Anandamid werden juckreizstillen Effekte beschrieben, die durch die Hemmung seines Abbaues verstärkt werden (Tey et al., 2011).

Die Untersuchung des Paradieskörner-Extraktes auf eine mögliche inhibitorische Wirkung auf das FAAH-Enzym erfolgte mit dem Fatty Acid Amide Hydrolase Inhibitor Screening Assay von Cayman (Item No. 10005196). Als Positivkontrolle diente CAY10435 von Cayman (Item No. 10005102). Die Testsubstanzen wurden in einer Endkonzentration von 30 µg/ml und 0,1 % DMSO eingesetzt und CAY10435 in einer Konzentration von 100 nM in 0,1 % DMSO. Die Durchführung und Handhabung des Assays erfolgte nach dem Protokoll des Herstellers. Die Messung der Fluoreszenz erfolgte im Fluoreszenzphotometer (spectraMAX Gemini).

Eine Zusammenstellung der Ergebnisse findet sich in Tabelle 2. Aus den Ergebnissen ist ersichtlich, dass erfindungsgemäße Extrakte aus Aframomum eindeutig die enzymatische Aktivität des Enzyms FAAH hemmen.

**Tabelle 2: Hemmung des Enzyms FAAH**

| Testsubstanz | Konzentration | Ergebnis (% Veränderung gegenüber Kontrolle) |
|---|---|---|
| Erfindungsgemäßer Extrakt Bsp. 1 | 30 µg/ml | -89,62 |
| Erfindungsgemäßer Extrakt Bsp. 2 | 30 µg/ml | -84,25 |
| CAY10435 | 100 nM | -86,68 |
| DMSO Kontrolle | 0,1% | 0 |

### Beispiele

### Beispiel 1: Erfindungsgemäßer Trockenextrakt aus Samen von Aframomum melegueta

100 g gemahlene Samen von Aframomum melegueta wurden zweimal mit der jeweils siebenfachen Menge 70 Gew.-% Ethanol 1h bei 60°C Wasserbadtemperatur am Rotationsverdampfer extrahiert. Die Extraktlösung wurde über ein Seitz Filter 1500 abgesaugt und am Rotationsverdampfer von Ethanol befreit. Der Rückstand wurde mit 2-Butanon-gesättigtem Wasser (27,1% 2-Butanon) auf einen Trocknungsrückstand (TR) von 10% (m/m) eingestellt. Die Lösung wurde dreimal mit der 2,5fachen Gewichtsmenge bezogen auf den TR in der Wasserphase, gegen die Mischung aus 84% 2-Butanon/ 10% Heptan/ 6% Wasser (m/m) ausgeschüttelt.

Die vereinigten organischen Phasen wurden am Rotationsverdampfer bei 50°C und reduziertem Druck zur Trockene eingedampft. Der Extrakt wurde im Trockenschrank bei 50°C und 12 mbar für 20 h nachgetrocknet: 4,34 g (4,34 %).

### Beispiel 2: Erfindungsgemäßer Trockenextrakt aus Samen von Aframomum melegueta

100 g gemahlene Samen von *Aframomum melegueta* wurden zweimal mit der jeweils siebenfachen Menge 60 Gew.-% Ethanol 1h bei 60°C Wasserbadtemperatur am Rotationsverdampfer extrahiert. Die Extraktlösung wurde über ein Seitz Filter 1500 abgesaugt und am Rotationsverdampfer von Ethanol befreit. Der Rückstand wurde dreimal mit der 2,5-fachen Gewichtsmenge bezogen auf den TR in der Wasserphase, gegen Ethylacetat ausgeschüttelt.

Die vereinigten organischen Phasen wurden am Rotationsverdampfer bei 50°C und reduziertem Druck zur Trockene eingedampft. Der Extrakt wurde im Trockenschrank bei 50°C und 12 mbar für 20 h nachgetrocknet: 3,90 g (3,90 %).

### Beispiel 3: Hydrophiles Tensidgel enthaltend Aframomum-Extrakt

Ein Trockenextrakt aus Samen von Aframomum melegueta (erfindungsgemäßer Extrakt gemäß Beispiel 1) wird mit Hilfsstoffen gemischt und in ein hydrophiles Tensidgel eingearbeitet.

| Pos. | Bestandteil | Mengenanteil (%) |
|---|---|---|
| 1 | Erfindungsgemäßer Aframomum-Extrakt nach Bsp. 1 | 1,0 |
| 2 | Macrogol-Glycerol-Hyd roxystearat | 35,0 |
| 3 | Isopropylmyristat | 10,0 |
| 4 | Neutralöl | 10,0 |
| 5 | Propylenglykol | 5,0 |
| 6 | Gereinigtes Wasser | 39,0 |

### Herstellung:

Position 1 (erfindungsgemäßer Aframomum-Extrakt), Position 2 (Tensid; Macrogol-Glycerol-Hydroxystearat), Position 3 (Isopropylmyristat), Position 4 (Neutralöl) und Position 5 (Propylenglykol) werden homogen gemischt. Anschließend wird das Wasser (Position 6) unter Rühren zugefügt. Es bildet sich ein homogenes Gel. Zu dieser Basisrezeptur können pharmazeutisch und kosmetisch gebräuchliche Stabilisatoren wie z.B. Ascorbinsäure oder Tocopherolacetat hinzugefügt werden.

### Beispiel 4: Wässriges Ethylcellulose-Gel enthaltend Aframomum-Extrakt

Ein Trockenextrakt aus Samen von Aframomum melegueta (erfindungsgemäßer Extrakt gemäß Beispiel 1) wird mit Hilfsstoffen gemischt und in ein wässriges Ethylcellulose-Gel eingearbeitet.

| Pos. | Bestandteil | Mengenanteil (%) |
|---|---|---|
| 1 | Erfindungsgemäßer Aframomum-Extrakt nach Bsp.1 | 0,5 |
| 2 | Polidocanol (Laureth 9) | 0,5 |
| 3 | Hydroxyethylcellulose | 2,5 |
| 4 | Glycerol 85 % | 7,0 |
| 5 | Xylitol-Lösung 10 % in Wasser | 10,0 |
| 6 | Kaliumsorbat | 0,1 |
| 7 | Gereinigtes Wasser | 79,4 |

### Herstellung:

Position 1 (erfindungsgemäßer Aframomumextrakt) wird mit dem Emulgator (Position 2; Polidocanol) und dem Gelbildner (Position 3; Hydroxyethylcellulose) gemischt. Die Mischung wird im Feuchthaltemittel (Position 4, Glycerol 85 %) dispergiert. Das Konservierungsmittel (Position 6; Kaliumsorbat) wird in einer Teilmenge des gereinigten Wassers (Position 7) gelöst und die wässrige Xylitol-Lösung (Position 5) zugefügt. Diese Lösung wird mit dem restlichen Wasser vereinigt. Die Mischung aus Positionen 1 - 4 (Extrakt, Emulgator, Gelbildern und Glycerol) wird in mehreren Schritten mit der wässrigen Phase vereinigt. Es bildet sich ein leicht getrübtes homogenes Gel.

### Beispiel 5: Lotion enthaltend Aframomum-Extrakt

Ein Trockenextrakt aus Samen von Aframomum melegueta (erfindungsgemäßer Extrakt gemäß Beispiel 1) wird mit Hilfsstoffen gemischt und in eine Lotion eingearbeitet.

| Pos. | Bestandteil | Mengenanteil (%) |
|---|---|---|
| 1 | Erfindungsgemäßer Aframomum-Extrakt nach Bsp.1 | 0,5 |
| 2 | Glycerinmonostearat | 2,0 |
| 3 | Cetylalkohol | 2,0 |
| 4 | Neutralöl | 15,0 |
| 5 | Mandelöl | 5,0 |
| 6 | Lecithin | 2,0 |
| 7 | Glycerol | 5,0 |
| 8 | Xanthan Gummi | 0,3 |
| 9 | Benzylalkohol | 2,0 |
| 10 | Gereinigtes Wasser | 39,0 |

### Herstellung:

Positionen 2-5 werden bei ca. 70°C geschmolzen (= lipophile Phase). Der Wirkstoff (Position 1) wird in der lipophilen Phase dispergiert. Position 6 (Lecithin) wird aufgestreut und homogen eingearbeitet. Positionen 7 (Glycerol) und 9 (Benzylakohol) werden im Wasser (Position 10) gelöst (= hydrophile Phase). Position 8 (XanthanGummi) wird unter intensivem Rühren in die Wasserphase homogen eingearbeitet. Die lipophile Phase und die hydrophile Phase werden unter Rühren vereinigt und homogenisiert. Es bildet sich eine homogene, beigefarbene flüssige Emulsion (= Lotion).

## Patentansprüche

1. Verfahren zur Herstellung eines Extraktes aus Samen von *Aframomum melegueta,* **gekennzeichnet durch** folgende Verfahrensschritte in der angegebenen Reihenfolge:
(a) Extraktion von getrockneten und gemahlenen Samen von *Aframomum melegueta* mit einem organischen Lösungsmittel oder mit einem Gemisch aus einem oder mehreren organischen Lösungsmittel und Wasser oder mit Wasser bei einer Temperatur zwischen 10 °C und 100 °C, um ein Gemisch aus extrahiertem Pflanzenmaterial und einer Extraktlösung zu erhalten, anschließend
(b) Trennung des ausextrahierten Pflanzenmaterials von der Extraktlösung, anschließend
(c) ggf. erneute Extraktion des in Schritt (b) erhaltenen, ausextrahierten Pflanzenmaterials mit einem Lösungsmittel gemäß Schritt (a) und Trennung gemäß Schritt (b), anschließend
(d) Vereinigung der in den Schritten (b) und (c) gewonnenen Extraktlösungen, nachfolgend
(e) Eindampfen der vereinigten Lösung aus Schritt (d), um einen Eindampfrückstand zu erhalten und Flüssig-flüssig-Verteilung des erhaltenen Eindampfrückstands zwischen Wasser und einem nicht mit Wasser mischbaren organischen Lösungsmittel ausgewählt aus 1-Butanol, 2-Butanon und einem Gemisch aus 2-Butanon und Heptan, um eine wässrige und eine organische Phase zu erhalten,
(f) Trennung der erhaltenen wässrigen und der organischen Phase aus Schritt (e)
(g) Eindampfen und Trocknen der aus Schritt (f) erhaltenen organischen Phase zum Trockenextrakt.

2. Verfahren nach Anspruch 1, wobei in Schritt (a) als organisches Lösungsmittel Alkohole oder Ketone sowie deren Gemische mit Wasser verwendet werden.

3. Verfahren nach Anspruch 2, wobei der Alkohol Ethanol und das Keton Aceton ist.

4. Verfahren nach Anspruch 3, wobei Ethanol und Wasser bzw. Aceton und Wasser in einem Mischungsverhältnis von 20 / 80 bis 80 / 20 (Gew./Gew.) eingesetzt werden.

5. Verfahren nach Anspruch 3, wobei Ethanol und Wasser bzw. Aceton und Wasser in einem Mischungsverhältnis von 50 / 50 bis 70 / 30 (Gew./Gew.) eingesetzt werden.

6. Extrakt aus Samen von *Aframomum melegueta* erhältlich nach dem Verfahren gemäß einem der Ansprüche 1 bis 5.

7. Extrakt nach Anspruch 6 zur Verwendung zur Beseitigung oder Linderung von akutem oder chronischem Juckreiz.

8. Extrakt zur Verwendung gemäß Anspruch 7, wobei der Extrakt topisch verabreicht wird und wobei der Juckreiz hervorgerufen werden kann durch Neurodermitis, ein allergisches Kontaktekzem, allergische Hautausschläge, Nesselsucht, Infektionserkrankungen, Insektenstiche, Parasitenbefall, geschädigte, stark belastete Haut, die extrem trocken ist, oder Krebserkrankungen.

9. Pharmazeutische oder kosmetische Zubereitung, enthaltend einen Extrakt nach Anspruch 6 und geeignete Hilfsstoffe als topische Darreichungsform, ausgewählt aus einem hydrophilen Tensidgel, einem Ethylcellulose-Gel und einer Lotion.

10. Pharmazeutische oder kosmetische Zubereitung nach Anspruch 9, wobei als Hilfsstoffe für das hydrophile Tensidgel Macrogol-Glycerol-Hydroxystearat, Isopropylmyristat, Neutralöl, Propylenglykol und gereinigtes Wasser, für das Ethylcellulose-Gel Polidocanol, Hyroxyethylcellulose, Glycerol 85%, Xylitol-Lösung 10 % in Wasser, Kaliumsorbat und gereinigtes Wasser und für die Lotion Glycerinmonostearat, Cethylalkohol, Neutralöl, Mandelöl, Lecithin, Glycerol, Xanthan Gummi, Benzylalkohol und gereinigtes Wasser verwendet werden.

## Claims

1. A method for producing an extract from seeds of *Aframomum melegueta*, **characterized by** the following method steps in the given order:
(a) extraction of dried and ground seeds of *Aframomum melegueta* with an organic solvent or with a mixture of one or several organic solvents and water or with water at a temperature between 10 °C and 100 °C in order to obtain a mixture of extracted plant material and an extract solution, followed by
(b) separation of the extracted plant material and the extract solution, followed by
(c) optionally another extraction of the extracted plant material obtained in step (b) with a solvent according to step (a) and separation according to step (b), followed by
(d) combination of the extraction solutions obtained in the steps (b) and (c), followed by
(e) evaporating the combined solution from step (d) in order to obtain an evaporation residue and liquid-liquid-distribution of the obtained evaporation residue between water and an organic solvent, which is not water-miscible, selected from 1-butanol, 2-butanone, and a mixture of 2-butanone and heptane in order to obtain an aqueous and an organic phase,
(f) separation of the obtained aqueous and organic phase of step (e)
(g) evaporating and drying the organic phase obtained in step (f) for a dry extract.

2. A method according to claim 1, wherein in step (a) alcohols or ketones as well as their mixtures with water are used as organic solvent.

3. A method according to claim 2, wherein the alcohol is ethanol and the ketone is acetone.

4. A method according to claim 3, wherein ethanol and water, respectively acetone and water, are used in a mixing ratio of 20/80 to 80/20 (wt./wt.).

5. A method according to claim 3, wherein ethanol and water, respectively acetone and water, are used in a mixing ratio of 50/50 to 70/30 (wt./wt.).

6. An extract from seeds of *Aframomum melegueta,* obtainable by the method according to one of claims 1 to 5.

7. An extract according to claim 6 for use in eliminating or relieving of acute or chronical itchiness.

8. An extract for use according to claim 7, wherein the extract is administered topically and wherein the itchiness can be caused by atopic dermatitis, allergic contact dermatitis, allergic rashes, nettle (rash), infectious diseases, insect bites, parasite attack, damaged, heavily stressed skin that is extremely dry, metabolic disorders, or cancerous diseases.

9. A pharmaceutical or cosmetic preparation containing an extract according to claim 6 and suitable excipients, as a topical dosage form selected from a hydrophilic tenside gel, an ethyl cellulose gel, and a lotion.

10. A pharmaceutical or cosmetic preparation according to claim 9, wherein macrogol glycerol hydroxyl stearate, isopropyl myristate, neutral oil, propylene glycol, and purified water are used as excipients for the hydrophilic tenside gel, wherein polidocanol, hydroxyl ethyl cellulose, glycerol 85 %, xylitol solution 10 % in water, potassium sorbate, and purified water are used as excipients for the ethyl cellulose gel, and wherein glycerol monostearate, cethyl alcohol, neutral oil, almond oil, lecithin, glycerol, xanthan gum, benzyl alcohol, and purified water are used as excipients for the lotion.

## Revendications

1. Procédé de production d'un extrait de graines d'Aframomum melegueta, **caractérisé par** les étapes de procédé suivantes dans l'ordre indiqué:
(a) l'extraction de graines séchées et broyées d'Aframomum melegueta avec un solvant organique ou avec un mélange d'un ou de plusieurs solvant (s) organique (s) et de l'eau ou avec de l'eau à une température comprise entre 10°C et 100°C, pour obtenir un mélange de matière végétale extraite et d'une solution d'extrait, puis
(b) la séparation de la matière végétale extraite et de la solution d'extrait, puis
(c) éventuellement nouvelle extraction de la matière végétale extraite obtenue à l'étape (b) avec un solvant selon l'étape (a) et séparation selon l'étape (b), puis
(d) combinaison des solutions d'extraits obtenues aux étapes (b) et (c), puis
(e) évaporation de la solution combinée de l'étape (d) pour obtenir un résidu d'évaporation et partage liquide-liquide du résidu d'évaporation obtenu entre l'eau et un solvant organique non miscible à l'eau, choisi parmi le butanol-1, le 2-butanone ou un mélange de 2-butanone et d'heptane, pour obtenir une phase aqueuse et une phase organique;
(f) la séparation de la phase aqueuse et de la phase organique, qui sont obtenues à l'étape (e);
(g) l'évaporation et le séchage de la phase organique obtenue à l'étape (f) pour obtenir un extrait sec.

2. Procédé selon la revendication 1, dans lequel à l'étape (a) des alcools ou des cétones et leurs mélanges avec de l'eau sont utilisés comme solvant organique.

3. Procédé selon la revendication 2, dans lequel l'alcool est l'éthanol et la cétone est l'acétone.

4. Procédé selon la revendication 3, dans lequel l'éthanol et l'eau ou l'acétone et l'eau sont utilisés dans un rapport de mélange de 20/80 à 80/20 (poids/poids).

5. Procédé selon la revendication 3, dans lequel l'éthanol et l'eau ou l'acétone et l'eau sont utilisés dans un rapport de mélange de 50/50 à 70/30 (poids/poids).

6. Extrait de graines d'Aframomum melegueta pouvant être obtenu par le procédé selon l'une des revendications 1 à 5.

7. Extrait selon la revendication 6 pour l'utilisation pour éliminer ou soulager les démangeaisons aiguës ou chroniques.

8. Extrait pour l'utilisation selon la revendication 7, dans lequel l'extrait est administré par voie topique, et les démangeaisons peuvent être causées par la neurodermite, une dermatite de contact allergique, des éruptions cutanées allergiques, l'urticaire, des maladies infectieuses, des piqûres d'insectes, une attaque de parasite, une peau endommagée et fortement stressée, qui est extrêmement sèche, ou des maladies cancéreuses.

9. Préparation pharmaceutique ou cosmétique, contenant un extrait selon la revendication 6 et des excipients appropriés, sous forme posologique topique choisie parmi un gel tensioactif hydrophile, un gel d'éthylcellulose et une lotion.

10. Préparation pharmaceutique ou cosmétique selon la revendication 9, dans laquelle l'hydroxystéarate de macrogol glycérol, l'huile neutre, le propylène glycol et l'eau purifiée sont utilisés comme excipients pour le gel tensioactif hydrophile; le Polidocanol, l'hydroxyéthylcellulose, le glycérol 85%, une solution de xylitol 10% dans l'eau, le sorbate de potassium et l'eau purifiée sont utilisés comme excipients pour le gel d'éthylcellulose, et le monostéarate de glycérol, l'alcool céthylique, l'huile neutre, l'huile d'amande, la lécithine, le glycérol, la gomme de xanthane, l'alcool benzylique et l'eau purifiée sont utilisés comme excipients pour la lotion.
